## Europäisches Patentamt
## European Patent Office
## Office européen des brevets

(11) Publication number: **0 146 514**
**B1**

(12) **EUROPEAN PATENT SPECIFICATION**

(45) Date of publication of patent specification: **21.12.88**

(51) Int. Cl.⁴: **G 01 N 15/08,** G 01 N 33/46

(21) Application number: **84850367.8**

(22) Date of filing: **28.11.84**

(54) **Method and device for measuring the gas and/or liquid permeability in the surface layer of an object.**

(30) Priority: **20.12.83 SE 8307045**

(43) Date of publication of application:
**26.06.85 Bulletin 85/26**

(45) Publication of the grant of the patent:
**21.12.88 Bulletin 88/51**

(84) Designated Contracting States:
**DE FR GB IT NL SE**

(56) References cited:
**GB-A-1 197 631**
**US-A-3 861 196**
**US-A-3 889 521**
**HOLZ ALS ROH- UND WERKSTOFF, vol. 35,
1977, pages 29-39, Berlin; G. BÖHNER "Zur
Wasserdampf- und Luftdurchlässigkeit
verschiedener unbehandelter und thermisch
behandelter Hölzer", Teil 2**
**HOLZ ALS ROH- UND WERKSTOFF, vol. 34,
1976, pages 295-307, Berlin; G. BÖHNER "Zur
Wasserdampf- und Luftdurchlässigkeit
verschiedener unbehandelter und thermisch
behandelter Hölzer", Teil 1**

(73) Proprietor: **TRÄTEKNIKCENTRUM
Drottning Kristinas väg 67
S-114 86 Stockholm (SE)**

(72) Inventor: **Boutelje, Julius Berthold
Skansvägen 44
S-191 43 Sollentuna (SE)**
Inventor: **Hägglund, Bengt Göran
Hemmansgatan 264
S-931 56 Skelleftea (SE)**

(74) Representative: **Illum, Leif-Otto
Svenska Cellulosa Aktiebolaget SCA
Kungsgatan 33
S-111 56 Stockholm (SE)**

Courier Press, Leamington Spa, England.

# EP 0 146 514. B1

**Description**

This invention relates to a method of measuring the gas and/or liquid permeability in the surface layer of an object. The invention particularly refers to a method of identifying timber damaged by wet storage. The invention also refers to a device for carrying out the method.

The invention is based on the problem that it is not possible to visually distinguish timber with wet storage damage from timber without such damage. The problem has been emphasized in investigations, which were carried out on timber with the object of determining which disadvantages are involved in wet storage from an application-technical aspect. Timber, which is not sawn before the warm season, must be wet stored in order to prevent crack formation, blue, insect attack and other damages. It is generally known, however, that wet storage during the warm season causes an increase in the permeability of the timber for liquids. This increase in permeability is of disadvantage in different connections and can have the effect, that the timber cannot be used for certain purposes, for example glazing.

Great volumes of timber are wet stored during the warm season. The storage time for the greatest part of wet stored timber is so long, that there are serious risks of increased permeability—"wet storage damages".

Timber with wet storage damages cannot be distinguished visually from timber without such damages, neither prior nor subsequent to drying. The object of the development work, which has resulted in the invention, therefore, had been to develop a method and an equipment for identifying timber damaged by wet storage.

According to US Patent 3,861,196 there is prior known an apparatus for measuring the quantity of a liquid e.g. water which passes through, in a unit of time, from one part of the other of a predetermined stratum of material. The stratum may be of board covered in bituminous conglommerate. The apparatus comprises a central chamber, adapted to hold measuring liquid, said chamber being surrounded by an annular chamber in which is contained a fluid, for example a gas, at normal pressure or greater than the pressure in the central chamber, thereby preventing the liquid in the central chamber from flowing outwardly along the surface. In this method measuring fluid is retained n the central chamber by the annular chamber.

In U.K. Patent 1,197,631 there is disclosed an apparatus for measuring the porosity of constructional materials, for example concrete pressure vessels. The apparatus comprise an inner and outer chamber. By creating a balanced vacuum between the inner and outer chamber, air is prevented from leaking into the inner chamber. The apparatus can only be used for measurement under vacuum.

The invention is based on the idea that a gas via a nozzle abutting the surface of an object is pressed into the object, and that the gas flow indicates the permeability of the object. A modification of this basic idea is to use vacuum instead of overpressure and to measure the air flow sucked out of the object. Another modification is to use liquid instead of gas as measuring medium.

The invention is described in greater detail in the following by way of embodiment thereof and with reference to the accompanying drawings, in which Figure 1 is a schematic diagram of an arrangement according to the invention, and Figures 2 and 3 show two different embodiments of the nozzle used according to the invention.

The air pressure required for obtaining the measuring pressure and, respectively, contact pressure for the nozzle is delivered from a compressor 1 or other pressure source. The desired measuring pressure is adjusted by means of reducing valves 3 and 5, and air flows into the system when the valve 2 is opened. The pressure before an air filter 7 is read by a pressure transducer 4. Upon opening a valve 6 the air continues to flow via an air filter 7 to an electric flow meter 8, which is connected to a multi-point printer (not shown). The air pressure after the flow meter (the measuring pressure) is recorded continuously by a pressure transducer 9 connected to a multi-point printer. The air is passed through a valve 10 to the nozzle 12. For venting the system, the valve 11 is opened.

The contact pressure for the nozzle 12 against the test specimen 13 is effected by using a compressed-air piston 19 with air from the compressed-air source 1 via the valves 2, 3, 20 and 15. The contact force is adjusted by reducing valves 3 and 20 and is read/recorded by pressure transducer 14. This arrangement implies that the measuring pressure and the contact force of the nozzle can be adjusted independently of each other. The system is vented by a valve 16 for rendering lifting of the nozzle 12 possible.

Compressed air from the source 1 is used also for lifting the nozzle 12 from the test specimen. This is effected through a conduit via the valve 17. The system can be vented through a valve 18.

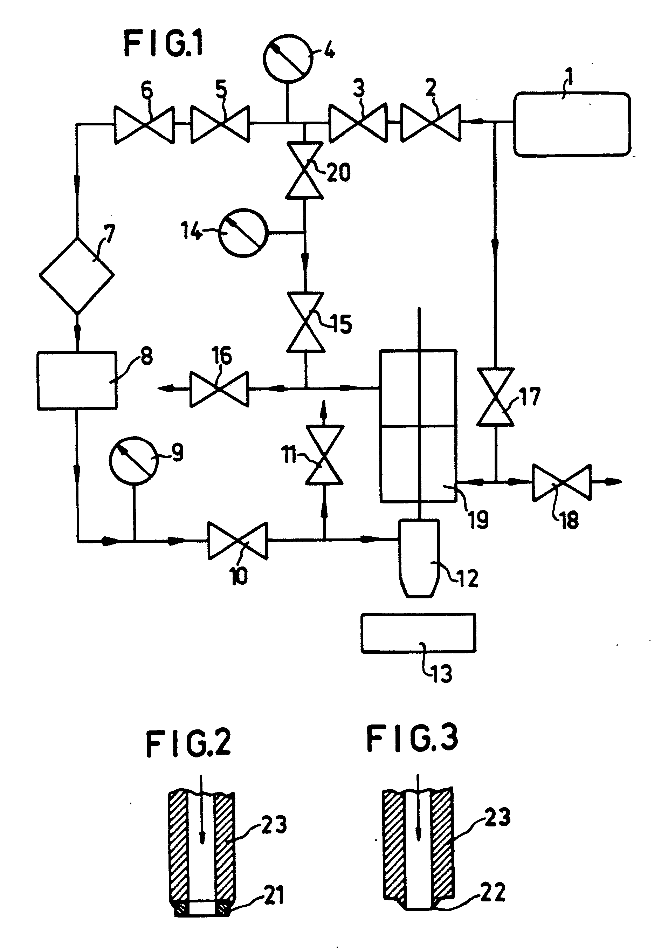

At the measurements carried out two different types of nozzle have been used, which can be exchanged against each other (Figures 2 and 3). According to the embodiment shown in Figure 2, the nozzle consists of a cylinder 23, which downwardly has a flange for inserting a gasket 21 therein. The gasket is so designed and of such a material, that it yields at a given contact pressure good sealing against the test specimen 13. The embodiment according to Figure 3 shows a so-called punch nozzle, at which the cylinder 23 at its lower edge is provided with a sharp edge 22, which is pressed-in slightly into the test specimen and thereby brings about the desired sealing against said specimen.

When the equipment described is used for measuring the gas permeability in the surface layer of timber in order thereby to determine the occurrence of wet storage damages, the nozzle 12 is positioned

2

# EP 0 146 514 B1

against the timber surface. When the valve 2 is opened, the nozzle 12 attached to a compressed-air piston 19 is pressed by air pressure against the timber 13. Upon opening the valve 5, the air flows via an electric flow meter 8 connected to a multi-point printer into the nozzle and into the timber. The measuring pressure and flow are recorded on the printer. After the measurement, the valves 2, 10 and 15 are closed, and the valves 11 and 16 are opened for venting the nozzle 12 and compressed-air cylinder 19. Thereafter the reducing valve 17 is opened for lifting the nozzle from the test specimen. A new test place or a new test specimen can now be measured. For unplaned timber the punch nozzle according to Figure 3 is used, for planed timber the nozzle with sealing gasket according to Figure 2 is used.

The contact force of the nozzle is proportional to the air pressure acting on the compressed-air piston. At the majority of measurements the contact force of the nozzle was about 392 N.

The flow meter used at the tests has a measuring range of 0—10 l/min. The error in measurement is estimated to be about ±0,1 l/min. This implies that the relative error in measurement at small air flows, for example at flows below 0,5 l/min, is quite essential. Problems of this kind can be eliminated by inserting into the conduit system an additional flow meter with a smaller measuring range and lower error in measurement.

Table 1 shows the flow values obtained for different types of wood. The Table shows substantial differences between timber with and without wet storage damages, between pine and spruce sapwood and between pine sapwood and pine heartwood. The values in Table 1 refer to room dry wood.

TABLE 1
Air flow at 2,5 atmosphere gauge measuring
pressure for different wood types

| Wood type | Air flow l/min |
|---|---|
| Pine sapwood with wet storage damages | 1,6—8,7 |
| Pine sapwood, not wet stored | 0,3 |
| Pine heartwood, not wet stored | <0,1 |
| Spruce sapwood with wet storage damages | 1,5 |
| Spruce sapwood and heartwood, not wet stored | <0,1 |

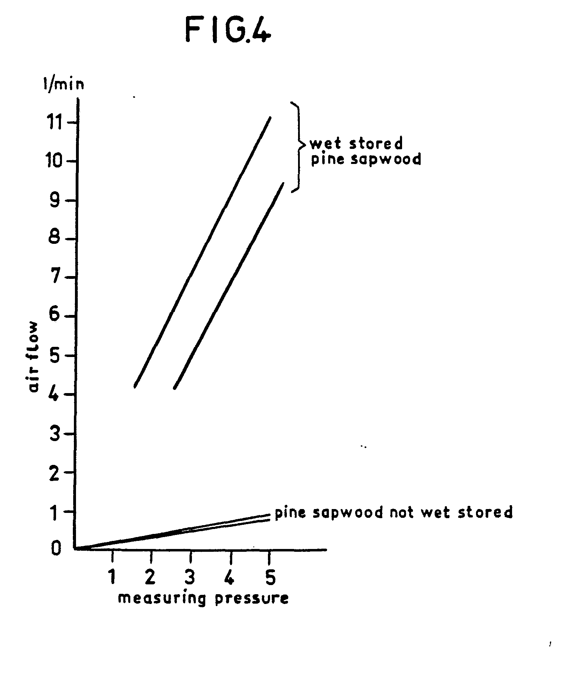

The air flow at pine sapwood with wet storage damages was 5—30 times greater than at pine sapwood without such damages. Pine sapwood with wet storage damages, thus, can easily be distinguished from pine sapwood without such damages. From Table 1 appears also the possibility to distinguish, for example, pine and spruce sapwood. Figure 4 shows that there is a linear relation between air flow and measuring pressure, and that the difference in air flow between timber with and without wet storage damages is greater the higher measuring pressure is used. Measuring pressures of up to 5 atmospheric gauge have been used, but it is fully possible to use higher measuring pressures.

The invention does not imply special requirements on the dimensions of the test specimen. It also is possible to carry out measurements on a construction in situ.

The invention is not restricted to the embodiment shown, but can be varied within the scope of the invention as claimed in the claims. It is, for example, possible to connect in parallel two flow meters with different measuring ranges. The object of such arrangement is that in cases when the flow is too small for one flow meter, the second one intended for smaller flows can be switched in. When the invention is applied on an industrial scale, the valves can be controlled via a microprocessor.

## Claims

1. A method of non-destructive measurement of the gas permeability of the surface layer of a wood object for the purpose of identifying wet storage damages, characterized in that a nozzle is placed in contact with the surface of the object with such force, known as the contact force, that leakage of gas between the edge of the nozzle and the surface is minimized, that the gas in the nozzle is at an overpressure, known as the measuring pressure, that gas is caused to pass from said nozzle through parts of the surface layer of the object, that the contact force is regulated and the measuring pressure is measured and regulated, and finally that flow or the time-dependent change in measuring pressure and/or flow is used as a measure of the gas permeability of the surface layer of the object.

2. A method as defined in Claim 1, characterized in that the measuring pressure and the contact force of the nozzle are regulated and measured independently of each other.

3. A device for carrying out the method defined in Claims 1 and 2, characterized in that it includes a replaceable nozzle (12) intended for placing in contact with the surface of an object (13), a source of pressure (1), connected to the nozzle via valves (2, 3, 5, 6, 10) to provide the required pressure and flow of gas to the nozzle, pressure gauges (14 and 9) and valves (2, 3, 15, 20 and 2, 3, 5, 6, 10) for measurement/regulation of contact force and measuring pressure, and a flow meter (8) for measurement of gas flow.

4. A device as defined in Claim 3, characterized in that the contact force and the measuring pressure are measured and regulated independently of each other by placing the pressure gauge (14) and the valves (20, 15) in one branch of the gas flow and the pressure gauge (9) and valves (5, 6, 10) in a second branch of the gas flow.

5. A device as defined in Claim 3, characterized in that the gas flow is measured/recorded by means of a flow meter (8), for example, an electrical flow meter connected to a data collection unit.

6. A device as defined in Claim 3, characterized in that the nozzle (12) comprises a sharp edge for placing in contact with the surface of the object (13).

7. A device defined by Claim 3, characterized in that the nozzle (12) is provided with a gasket for sealing the contact against the object (13).

8. A device as defined in Claim 3, characterized in that it includes valves (11, 16, 18) used for venting the tube system connecting to the nozzle and the pneumatic air cylinder (19).

9. A device as defined in Claim 3, characterized in that the nozzle (12) is lifted from the surface of the object (13) by comprssed air supplied through valve (17) and a pneumatic piston.

## Patentansprüche

1. Verfahren zum zerstörungsfreien Messen der Gasdurchlässigkeit der Oberflächenschicht eines Holzgegenstandes zum Zweck der Erkennung von Beschädigungen durch feuchte Lagerung dadurch gekennzeichnet, daß eine Düse mit einer derartigen als Kontaktkraft bekannten Kraft in Berührung mit der Oberfläche des Gegenständes gebracht wird, daß das Austreten von Gas zwischen dem Rand der Düse und der Oberfläche so gering wie möglich ist, daß das Gas in der Düse unter einem Überdruck steht, der als Meßdruck bekannt ist, daß das Gas dazu gebracht wird, von der Düse durch Teile der Oberflächenschicht des Gegenstandes hindurchzugehen, daß die Kontaktkraft eingestellt wird und der Meßdruck gemessen und eingestellt wird, und daß schließlich die Strömung oder die zeitabhängige Änderung im Meßdruck und/oder der Strömung als Maß für die Gasdurchlässigkeit der Oberflächenschicht des Gegenstandes genommen wird.

2. Verfahren nach Anspruch 1, dadurch gekennzeichnet, daß der Meßdruck und die Kontaktkraft der Düse unabhängig voneinander eingestellt und gemessen werden.

3. Vorrichtung zur Durchführung des Verfahrens nach Anspruch 1 und 2, dadurch gekennzeichnet, daß eine austauschbare Düse (12) für die Anlage an der Oberfläche eines Gegenstandes (13) eine Druckquelle (1) die mit der Düse über Ventile (2, 3, 5, 6, 10) verbunden ist, um den erforderlichen Druck und die erforderliche Gasströmung der Düse zu liefern, Druckmesser (14 und 9) und Ventile (2, 3, 15, 20 und 2, 3, 5, 6, 10) zum Messen/Einstellen der Kontaktkraft und des Meßdruckes, und ein Strömungsmesser (8) zum Messen der Gasströmung vergesehen sind.

4. Vorrichtung nach Anspruch 3, dadurch gekennzeichnet, daß die Kontaktkraft und der Meßdruck unabhängig voneinander dadurch gemessen und eingestellt werden, daß der Druckmesser (14) und die Ventile (20, 15) in einem Zweig der Gasströmung und der Druckmesser (9) und die Ventile (5, 6, 10) in einem zweiten Zweig der Gasströmung angeordnet sind.

5. Vorrichtung nach Anspruch 3, dadurch gekennzeichnet, daß die Gasströmung mittels eines Strömungsmessers (8) beispielsweise eines elektrischen Strömungsmessers, gemessen und aufgezeichnet wird, der mit einer Datensammeleinheit verbunden ist.

6. Vorrichtung nach Anspruch 3, dadurch gekennzeichnet, daß die Düse (12) eine scharfe Kante zur Anlage an der Oberfläche des Gegenstandes (13) aufweist.

7. Vorrichtung nach Anspruch 3, dadurch gekennzeichnet, daß die Düse (12) mit einer Dichtung zum Abdichten der Anlage am Gegenstand (13) versehen ist.

8. Vorrichtung nach Anspruch 3, dadurch gekennzeichnet daß Ventile (11, 16, 18) zum Entlüften des Rohrleitungssystems vorgesehen sind, das die Düse und den pneumatischen Zylinder (19) verbindet.

9. Vorrichtung nach Anspruch 3, dadurch gekennzeichnet, daß die Düse (12) durch Druckluft, die über ein Ventil (17), geliefert wird, und einen pneumatischen Kolben von der Oberfläche des Gegenstandes (13) abgehoben wird.

## Revendications

1. Procédé pour la mesure non destructive de la perméabilité aux gaz de la couche superficielle d'un objet en bois aux fins d'identification des dommages dus au stockage humide, caractérisé en ce qu'une tuyère est placée en contact avec la surface de l'objet avec une force dite force de contact telle que les fuites de gaz entre le bord de la tuyère et la surface sont réduites au minimum, en ce que le gaz contenu dans la tuyère est soumis à une surpression dite pression de mesure, en ce que le gaz sortant de la tuyère traverse des parties de la couche superficielle de l'objet, en ce que la force de contact est réglée et que la pression de mesure est mesurée et réglée et enfin en ce que le débit ou la variation en fonction du temps de la pression de mesure et/ou du débit sont utilisés comme mesure de la perméabilité au gaz de la couche superficielle de l'objet.

2. Procédé selon la revendication 1, caractérisé en ce que la pression de mesure et la force de contact de la tuyère sont réglées et mesurées indépendamment l'une de l'autre.

3. Appareil pour la mise en oeuvre du procédé selon la revendication 1 ou 2, caractérisé en ce qu'il comprend une tuyère (12) remplaçable, conce pour être placée en contact avec la surface d'un objet (13), une source de pression (1) connectée à la tuyère par l'intermédiaire vannes (2, 3, 5, 6, 10) pour fournir la pression et le débit du gaz voulus à la tuyère, des indicateurs de pression (14 et 9) et des vannes (2, 3, 15, 20 et 2, 3, 5, 6, 10) pour la mesure et/ou le réglage de la force de contact et de la pression de mesure, et un débitmètre (8) pour la mesure du débit de gaz.

4. Appareil selon la revendication 3, caractérisé en ce que la force de contact et le pression de mesure sont mesurées et réglées indépendamment l'une de l'autre, du fait que l'indicateur de pression (14) et les vanns (20, 15) sont placés dans une branche du courant de gaz et que l'indicateur de pression (9) et les vannes (5, 6, 10) sont placés dans une seconde branche du courant de gaz.

5. Appareil selon la revendication 3, caractérisé en ce que le débit de gaz est mesuré et/ou enregistré au moyen d'un débitmètre (8), par exemple d'un débitmètre électrique connecté à un appareil pour la collecte de données.

6. Appareil selon la revendication 3, caractérisé en ce que la tuyère (12) comporte une arête vive qui est placée en contact avec la surface de l'objet (13).

7. Appareil selon la revendication 3, caractérisé en ce que la tuyère (12) est pourvue d'un garniture assurant un contact étanche avec l'objet (13).

8. Appareil selon la revendication 3, caractérisé en ce qu'il comporte des vannes (11, 16, 18) utilisées pour mettre à la pression ambiante le système de conduits assurant la liaison entre la tuyère et le cylindre pneumatique (19).

9. Appareil selon la revendication 3, caractérisé en ce que la tuyère (12) est écartée de la surface de l'objet (13) au moyen d'air comprimé fourni par la vanne (17) et par un piston pneumatique.

# FIG.1

# FIG.2

# FIG.3

# FIG.4